(19) **Europäisches Patentamt**

**European Patent Office**

**Office européen des brevets**

(11) **EP 1 369 104 A1**

(12) **EUROPEAN PATENT APPLICATION**

(43) Date of publication:
**10.12.2003 Bulletin 2003/50**

(51) Int Cl.[7]: **A61K 7/075**, A61K 7/08, A61K 7/11

(21) Application number: **03012563.7**

(22) Date of filing: **03.06.2003**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **08.06.2002 EP 02012764**

(71) Applicant: **Clariant GmbH
65929 Frankfurt am Main (DE)**

(72) Inventors:
• **Klug, Peter, Dr.
63762 Grossostheim (DE)**

• **Klein, Sonja
65795 Hattersheim (DE)**
• **Sandoval, Ana Maria
04602 002 Sao Paulo (BR)**

(74) Representative: **Paczkowski, Marcus, Dr. et al
Clariant Service GmbH
Patente, Marken, Lizenzen
Am Unisys-Park 1
65843 Sulzbach (DE)**

(54) **Single-phase cosmetic care compositions**

(57) A single-phase hair cosmetic preparation is disclosed, which comprises, as active substance, a compound of the formula

$$R(OCHR'CH_2)_w(OCHR''CH_2)\text{-}O(CH_2)_3\text{-}Me_2SiO(R'''MeSiO)_y(Me_2SiO)_zSiMe_2(CH_2)_3\text{-}O\text{-}$$

$$(CH_2CHR''O)_w(CH_2CHR'O)_xR,$$

where Me is methyl, R is an alkyl group having 1 to 4 carbon atoms, R' and R'', independently of one another, are hydrogen or an alkyl group having 1 to 4 carbon atoms, R''' is a linear or branched alkyl group having 4 to 18 carbon atoms, and x and w may be a number from 0 to 60, preferably 5 to 30, particularly preferably 10 to 20, y may be a number from 5 to 100 and z may be a number from 5 to 200, where the sum of the numbers x and w must be greater than or equal to 1 and the alkoxy groups may be in random distriburion or else be in block form, y may be a number from 5 to 100 and z may be a number from 5 to 200.

**Description**

The present invention relates to single-phase cosmetic preparations based on water, alcohol or oil for haircare, comprising alkylmethylsiloxane-dimethylsiloxane-polyalkylene oxide copolymers.

Frequent bleaching, permanent waving and coloring, but also frequent washing, of the hair using degreasing surfactants leads to damage of the hair structure. The hair becomes brittle and loses its shine. Roughened surfaces of the hair cause matting and knotting of the hair, and combability is impaired. Consequently, hair-treatment compositions which improve shine, wet combability and dry combability, condition and depth of color of the hair have achieved considerable importance. Moreover, haircare compositions aim, through shorter drying times, to reduce thermal stress to the hair during blow drying and to "repair" existing hair damage, such as, for example, split-ends.
The patent literature contains numerous proposals for realizing such intentions, including the use of water-soluble polymers, cationic fatty acid derivatives, mainly cationic, in particular quaternary ammonium compounds, such as cetyltrimethylammonium chloride, alone or in combination with various wax-like additives, such as, for example, hydrocarbons, fatty alcohols and fatty acids. Oils and oil-like substances, such as, for example, liquid hydrocarbon compounds, fatty alcohols, monocarboxylic acid esters, polyalcohol esters and silicones are also used.
A disadvantage of the above-described compositions is that, after they have been rinsed off, they often impart a sticky feel to damp hair, exhibit a "build-up" effect and weigh down dry hair or, particularly in the case of fine hair, make the hair so soft that it can barely still be styled.
In addition, unfavorable solubility behavior and incompatibilities of the active substances with other ingredients considerably limit the scope of formulation.

EP 1 149 872 describes the preparation of novel silicone co-polyols and praises their use as emulsifier for silicone-in-water emulsions and in oil-in-water emulsions with use possibilities in cosmetics, the textile industry, structural engineering and automotive engineering.

Surprisingly, it has been found that this novel class of alkylmethylsiloxane-dimethylsiloxane-polyalkylene oxide copolymers according to EP 1 149 872 have excellent conditioning effects for the hair and are valuable active substances in hair cosmetic, non-emulsion-like, i.e. single-phase, systems.
For example, these compounds bring about significantly better hair shine and easier combability compared with conventional silicones.
Advantages include good solubility in water, and also good compatibility with hydrophobic compounds, improved clarity of the composition, and favorable viscosity behavior of the silicone polyols used according to the invention.

The invention provides single-phase hair cosmetic preparations comprising, as active substance, a compound of the formula

$$R(OCHR'CH_2)_w(OCHR''CH_2)x-O(CH_2)_3-Me_2SiO(R'''MeSiO)_y(Me_2SiO)_zSiMe_2(CH_2)_3-O-$$

$$(CH_2CHR''O)_x(CH_2CHR'O)_wR, \qquad \text{(formula 1)}$$

where Me is methyl, R is an alkyl group having 1 to 4 carbon atoms, R' and R'', independently of one another, are hydrogen or an alkyl group having 1 to 4 carbon atoms, R''' is a linear or branched alkyl group having 4 to 18 carbon atoms, and x and w may be a number from 0 to 60, preferably 5 to 30, particularly preferably 10 to 20, y may be a number from 5 to 100 and z may be a number from 5 to 200, where the sum of the numbers x and w must be greater than or equal to 1. The alkoxy groups may be in random distribution or else be arranged in block form.

Preference is given to a caprylyl-bis-PEG/PPG 20/20 methyl ether dimethicone, as available under the trade name SilCare® Silicone 140M30 (Clariant), according to the formula 1 where R is methyl, R' is hydrogen, R'' is methyl, R''' is n-octyl, w = 20, x = 20, y = 25 and z = 75, and also to caprylyl-bis-PEG 10 methyl ether dimethicone according to the formula 1 where R is methyl, R' is hydrogen, R'' is methyl, R''' is n-octyl, w = 10, x = 0, y = 25 and z = 75.

The compounds of the formula 1 and the preparation thereof is described in EP 1 149 872.

Preferred embodiments are clear, viscous, aqueous, aqueous-alcoholic preparations or preparations based on oil in the form of fluids, gels, oils, foams and sprays. These are, for example, shampoos, hair conditioners, hair cures, hair rinses, volumizing spray, styling fluid, hair foam, hair gel, setting composition, hair spray, mousse, hair oils and split-end fluids.

The hair cosmetic preparations based on water or water/alcohol mixtures according to the invention comprise silicone polyols according to formula 1 in amounts by weight of from 0.01% to 30%, preferably 0.02% to 10%, particularly preferably 0.05% to 5%, based on the finished compositions.

Oil-based preparations according to the invention comprise silicone polyols according to formula 1 in the amounts by weight of from 0.1 to 80%, preferably 0.5 to 60%, particularly preferably 1 to 50%, based on the finished compositions.

Suitable alcohols which may be present in the preparations according to the invention are all monohydric or polyhydric alcohols. Preference is given to alcohols having 1 to 4 carbon atoms, such as ethanol, propanol, isopropanol, n-butanol, isobutanol, tert-butanol, glycerol, in particular propylene glycol, butylene glycol, hexylene glycol and mixtures of said alcohols. Further preferred alcohols are polyethylene glycols with a relative molecular mass below 2000. In particular, a use of polyethylene glycol with a relative molecular mass between 200 and 600 and of polyethylene glycol with a relative molecular mass between 400 and 600 is preferred.

The oil-based compositions according to the invention may comprise: hydrocarbon oils having linear or branched, saturated or unsaturated $C_7$-$C_{40}$-carbon chains, for example dodecane, isododecane, cholesterol, hydrogenated polyisobutylenes, docosanes, hexadecane, isohexadecane, paraffins and isoparaffins, and also triglycerides of animal and vegetable origin, for example beef tallow, pig fat, goose grease, perhydrosqualene, lanolin, sunflower oil, maize oil, soybean oil, rice oil, jojoba oil, babussu oil, pumpkin oil, grapeseed oil, sesame oil, walnut oil, apricot oil, macadamia oil, avocado oil, sweet almond oil, lady's smock oil, castor oil, olive oil, groundnut oil, rapeseed oil and coconut oil and synthetic oils, such as purcellin oil, linear and/or branched fatty alcohols and fatty acid esters, preferably Guerbet alcohols having 6 to 18, preferably 8 to 10, carbon atoms; esters of linear ($C_6$-$C_{13}$)-fatty acids with linear ($C_6$-$C_{20}$)-fatty alcohols; esters of branched ($C_6$-$C_{13}$)-carboxylic acids with linear ($C_6$-$C_{20}$)-fatty alcohols, esters of linear ($C_6$-$C_{18}$)-fatty acids with branched alcohols, in particular 2-ethylhexanol; esters of linear and/or branched fatty acids with polyhydric alcohols (such as, for example, dimerdiol or trimerdiol) and/or Guerbet alcohols; alcohol esters of $C_1$-$C_{10}$-carboxylic acids or $C_2$-$C_{30}$-dicarboxylic acids, esters, such as dioctyl adipate, diisopropyl dimer dilinoleate; propylene glycols/dicaprylate or waxes, such as beeswax, paraffin wax or microcrystalline waxes, optionally in combination with hydrophilic waxes, such as, for example, cetylstearyl alcohol; fluorinated and perfluorinated oils; Monoglycerides of $C_1$-$C_{30}$-carboxylic acids, diglycerides of $C_1$-$C_{30}$-carboxylic acids, triglycerides of $C_1$-$C_{30}$-carboxylic acids, for example triglycerides of caprylic/capric acids, ethylene glycol monoesters of $C_1$-$C_{30}$-carboxylic acids, ethylene glycol diesters of $C_1$-$C_{30}$-carboxylic acids, propylene glycol monoesters of $C_1$-$C_{30}$-carboxylic acids, propylene glycol diesters of $C_1$-$C_{30}$-carboxylic acids, and propoxylated and ethoxylated derivatives of the abovementioned classes of compound. The carboxylic acids may contain linear or branched alkyl groups or aromatic groups. Examples which may be mentioned are diisopropyl sebacate, diisopropyl adipate, isopropyl myristate, isopropyl palmitate, myristyl propionate, ethylene glycol distearate, 2-ethylhexyl palmitate, isodecyl neopentanoate, di-2-ethylhexyl maleate, cetyl palmitate, myristyl myristate, stearyl stearate, cetyl stearate, behenyl behenate, dioctyl maleate, dioctyl sebacate, cetyl octanoate, diisopropyl dilinoleate, caprylic/capryl triglyceride, PEG-6-caprylic/capryl triglyceride, PEG-8- caprylic/capryl triglyceride, cetyl ricinoleate, cholesterol hydroxystearate, cholesterol isostearate. $C_1$-$C_{30}$-Monoesters and polyesters of glycerol, for example glyceryl tribehenate, glyceryl stearate, glyceryl palmitate, glyceryl distearate, glyceryl dipalmitate.

$C_1$-$C_{30}$-Carboxylic acid monoesters and polyesters of sugars, for example glucose tetraoleate, glucose tetraester of soybean oil fatty acid, mannose tetraester of soybean oil fatty acid, galactose tetraester of oleic acid, arabinose tetraester of linoleic acid, xylose tetralinoleate, galactose pentaoleate, sorbitol tetraoleate, sorbitol hexaester of unsaturated soybean oil fatty acid, xylitol pentaoleate, sucrose tetraoleate, sucrose pentaoleate, sucrose hexaoleate, sucrose heptaoleate, sucrose oleate.

Available silicone oils are dimethylpolysiloxanes and cyclomethicones, polydialkylsiloxanes $R_3SiO(R_2SiO)_xSiR_3$, where R is a methyl and ethyl, particularly preferably methyl, and x is a number from 2 to 500, for example dimethicones available under the trade names VICASIL (General Electric Company), DOW CORNING 200, DOW CORNING 225, (Dow Corning Corporation).

Trimethylsiloxysilicates $[(CH_2)_3SiO_{1/2}]_x[SiO_2]_y$, where x is a number from 1 to 500 and y is a number from 1 to 500, Dimethiconols $R_3SiO[R_2SiO]_xSiR_2OH$ and $HOR_2SiO[R_2SiO]_xSiR_2OH$, where R is methyl or ethyl and x is a number up to 500, polyalkylarylsiloxanes, for example polymethylphenylsiloxanes available under the trade names SF 1075 METHYLPHENYL FLUID (General Electric Company) and 556 COSMETIC GRADE PHENYL TRIMETHICONE FLUID (Dow Corning Corporation), polydiarylsiloxanes, silicone resins, cyclic silicones and amino-, fatty acid-, alcohol-, polyether-, epoxy-, fluorine- and/or alkyl-modified silicone compounds, and also polyether siloxane copolymers.

The hair cosmetic compositions according to the invention can comprise, as further auxiliaries and additives, surfactants, cationic polymers, thickeners, film formers, and other additives customary in cosmetics, such as, for example, superfatting agents, moisture-donating agents, silicones, stabilizers, conditioning agents, glycerol, preservatives, pearlizing agents, dyes and fragrances, solvents, hydrotropic agents, opacifiers, fatty alcohols, substances with a keratolytic and keratoplastic action, and antidandruff agents.

Anionic washing-active substances which may be mentioned: $C_{10}$-$C_{20}$-alkyl and alkylene carboxylates, alkyl ether carboxylates, fatty alcohol sulfates, fatty alcohol ether sulfates, alkylamide sulfates and sulfonates, fatty acid alkylamide polyglycol ether sulfates, alkane sulfates, alkanesulfonates and hydroxyalkanesulfonates, olefinsulfonates, acyl esters of isethionates, α-sulfofatty acid esters, alkylbenzenesulfonates, alkylphenol glycol ether sulfonates, sulfosuccinates, sulfosuccinic monoesters and diesters, fatty alcohol ether phosphates, protein/fatty acid condensation products, alkyl monoglyceride sulfates and sulfonates, alkyl glyceride ethersulfonates, fatty acid methyltaurides, fatty acid sarkosinates, sulforicinoleates, amphoacetates or -glycinates, acyl glutamates. These compounds and mixtures thereof are used in the form of their water-soluble or water-dispersible salts, for example the sodium, potassium, magnesium, ammonium, mono-, di- and triethanolammonium and analogous alkylammonium salts.

Examples of suitable cationic surfactants are quaternary ammonium salts, such as di-($C_{10}$-$C_{24}$-alkyl)dimethylammonium chloride or bromide, preferably di-($C_{12}$-$C_{18}$-alkyl)dimethylammonium chloride or bromide; $C_{10}$-$C_{24}$-alkyldimethylethylammonium chloride or bromide; $C_{10}$-$C_{24}$-alkyltrimethylammonium chloride or bromide, preferably cetyltrimethylammonium chloride or bromide and $C_{20}$-$C_{22}$-alkyltrimethylammonium chloride or bromide; $C_{10}$-$C_{24}$-alkyldimethylbenzylammonium chloride or bromide, preferably $C_{12}$-$C_{18}$-alkyldimethylbenzylammonium chloride; N-($C_{10}$-$C_{18}$- alkyl) pyridinium chloride or bromide, preferably N-($C_{12}$-$C_{16}$-alkyl)pyridinium chloride or bromide; N-($C_{10}$-$C_{18}$-alkyl)isoquinolinium chloride, bromide or monoalkyl sulfate; N-($C_{12}$-$C_{18}$-alkylpolyoylaminoformylmethyl)pyridinium chloride; N-($C_{12}$-$C_{18}$-alkyl)-N-methylmorpholinium chloride, bromide or monoalkyl sulfate; N-($C_{12}$-$C_{18}$-alkyl)-N-ethylmorpholinium chloride, bromide or monoalkyl sulfate; $C_{16}$-$C_{18}$-alkylpentaoxethyl- ammonium chloride; diisobutylphenoxyethoxyethyldimethylbenzylammonium chloride; salts of N,N-diethylaminoethylstearylamide and -oleylamide with hydrochloric acid, acetic acid, lactic acid, citric acid, phosphoric acid; N-acylaminoethyl-N,N-diethyl-N-methylammonium chloride, bromide or monoalkyl sulfate and N-acylaminoethyl-N,N-diethyl-N-benzylammonium chloride, bromide or monoalkyl sulfate, where acyl is preferably stearyl or oleyl.

Examples of suitable nonionic surfactants which can be used as washing-active substances are: fatty alcohol ethoxylates (alkylpolyethylene glycols); alkylphenol polyethylene glycols; alkyl mercaptan polyethylene glycols; fatty amine ethoxylates (alkylaminopolyethylene glycols); fatty acid ethoxylates (acyl polyethylene glycols); polypropylene glycol ethoxylates (Pluronics® ); fatty acid amide polyethylene glycols; N-alkyl- and N-alkoxypolyhydroxy fatty acid amide, in particular fatty acid N-methylglucamides, sucrose esters; polyglycol ethers, alkylpolyglycosides, phosphoric esters (mono-, di- and triphosphoric esters ethoxylated and nonethoxylated).

Preferred amphoteric surfactants are: N-($C_{12}$-$C_{18}$-alkyl)-β-aminopropionates and N-($C_{12}$-$C_{18}$-alkyl)-β-iminodipropionates as alkali metal and mono-, di- and trialkylammonium salts; N-acylaminoalkyl-N,N-dimethylacetobetaine, preferably N-($C_8$-$C_{18}$-acyl)aminopropyl-N,N-dimethylacetobetaine; $C_{12}$-$C_{18}$-alkyldimethylsulfopropylbetaine; amphoteric surfactants based on imidazoline (trade name: Miranol® , Steinapon® ), preferably the sodium salt of 1-(β-carboxymethyloxyethyl)-1-(carboxymethyl)-2-laurylimidazolinium; amine oxides, e.g. $C_{12}$-$C_{18}$-alkyldimethylamine oxide, fatty acid amidoalkyldimethylamine oxide.

Furthermore, foam-boosting cosurfactants from the group consisting of alkylbetaines, alkylamidobetaines, aminopropionates, aminoglycinates, imidazoliniumbetaines and sulfobetaines, amine oxides and fatty acid alkanolamides or polyhydroxyamides can be used in the compositions according to the invention.

Preferred surfactants in the compositions according to the invention are lauryl sulfate, cocoamidopropylbetaine, sodium cocoylglutamate, disodium laureth sulfosuccinate and coco fatty acid diethanolamide.

The total amount of surfactants used in the compositions according to the invention can be between 5 and 70% by weight, preferably between 10 and 40% by weight, particularly preferably between 12 and 35% by weight, based on the finished composition.

Suitable cationic polymers are the compounds known under the INCI name "Polyquaternium", in particular Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, Polyquaternium 37&mineral oil&PPG trideceth (® Salcare SC95), PVP-dimethylaminoethyl methacrylate copolymer, Guar hydroxypropyltriammonium chlorides, and calcium alginate and ammonium alginate.

In addition, it is also possible to use cationic cellulose derivatives; cationic starch; copolymers of diallylammonium salts and acrylamides; quaternized vinylpyrrolidone/vinylimidazole polymers; condensation products of polyglycols and amines; quaternized collagen polypeptides; quaternized wheat polypeptides; polyethyleneimines; cationic silicone polymers, such as, for example, amidomethicones; copolymers of adipic acid and dimethylaminohydroxypropyldiethylenetriamine; polyaminopolyamide and cationic chitin derivatives, such as, for example, chitosan.

The desired viscosity of the compositions can be adjusted by adding thickeners. Suitable thickeners are cellulose ethers and other cellulose derivatives (e.g. carboxymethylcellulose, hydroxyethylcellulose), gelatins, starch and starch derivatives, sodium alginates, fatty acid polyethylene glycol esters, agar agar, tragacanth or dextrins.

The synthetic polymers used are various materials, such as, for example, polyvinyl alcohols, polyacrylamides, polyvinylamides, polysulfonic acids, in particular copolymers based on ammonium salts of acrylamidoalkylsulfonic acids and cyclic N-vinylcarboxamides or cyclic and linear N-vinylcarboxamides and also hydrophobically modified acrylamidoalkylsulfonic acid copolymers, polyacrylic acid, polyacrylic esters, polyvinylpyrrolidone, polyvinyl methyl ether, polyethylene oxides, copolymers of maleic anhydride and vinyl methyl ether, and various mixtures and copolymers of the abovementioned compounds, including their various salts and esters. These polymers can be crosslinked or uncrosslinked as desired.

Thickeners which are particularly suitable for oil-based compositions are dextrins, for example dextrin palmitate, but also fatty acid soaps, fatty alcohols and silicone waxes, for example the SilCare™ Silicone 41 M70 or SilCare™ Silicone 41 M80.

Depending on the intended use, suitable film formers are salts of phenylbenzimidazolesulfonic acid, water-soluble polyurethanes, for example $C_{10}$-polycarbamyl polyglyceryl ester, polyvinyl alcohol, polyvinylpyrrolidone and copolymers thereof, for example vinylpyrrolidone/vinyl acetate copolymer, water-soluble acrylic acid polymers/copolymers or esters or salts thereof, for example partial ester copolymers of acrylic/methacrylic acid and polyethylene glycol ethers of fatty alcohols, such as acrylate/steareth-20-methacrylate copolymer, water-soluble cellulose, for example hydroxymethylcellulose, hydroxyethylcellulose, hydroxypropylcellulose, water-soluble quaterniums, polyquaterniums, carboxyvinyl polymers, such as carbomers and salts thereof, polysaccharides, for example polydextrose and glucan, vinyl acetate/crotonate, available for example under the trade name Aristoflex A 60 (Clariant), and polymeric amine oxides, for example representatives available under the trade names Diaformer Z-711, 712, 731, 751 (Mitsubishi Chem.).

Further additives may be silicone compounds, for example dimethylpolysiloxane, methylphenylpolysiloxanes, cyclic silicones, and amino-, fatty acid-, alcohol-, polyether-, epoxy-, fluorine- and/ or alkyl-modified silicone compounds, for example Alkylsilicone SilCare® Silicone 41M10, SilCare® Silicone 41M15, SilCare® Silicone 41M20, SilCare® Silicone 41M30 (Clariant), Alkyltrimethicone SilCare™ 31M30, SilCare™ 31M40, SilCare™ 31 M 50, SilCare™ Silicone Caprylyl Trimethicone 31M60 (CLARIANT), SilCare™ Phenyl Trimethicone 15M30, SilCare™ 15M40, SilCare™ 15M50, SilCare™ 15M60, polyalkylarylsiloxanes and polyether siloxane copolymers.

Suitable carrier materials are vegetable oils, natural and hydrogenated oils, waxes, fats, water, alcohols, polyols, glycerol, glycerides, liquid paraffins, liquid fatty alcohols, sterol, polyethylene glycols, cellulose and cellulose derivatives.

Fungicides as active ingredients which may be used are ketoconazole, oxiconazole, terbinafine, bifonazole, butoconazole, cloconazole, clotrimazole, econazole, enilconazole, fenticonazole, isoconazole, miconazole, sulconazole, tioconazole, fluconazole, itraconazole, terconazole and naftifine, Zn pyrethione and octopyrox.

The compositions according to the invention can be mixed with conventional ceramides, pseudoceramides, fatty acid N-alkylpolyhydroxyalkylamides, cholesterol, cholesterol fatty acid esters, fatty acids, triglycerides, cerebrosides, phospholipids and similar substances.

Suitable pearlizing compounds are fatty acid monoalkanolamides, fatty acid dialkanolamides, monoesters or diesters of alkylene glycol, in particular of ethylene glycol and/or propylene glycol or oligomers thereof with higher fatty acids, e.g. palmitic acid, stearic acid or behenic acid or mixtures thereof. Mono- and diesters of alkylene glycols with fatty acids, fatty acids and metal salts thereof, monoesters or polyesters of glycerol with carboxylic acids and ketosulfones of various types. In the compositions according to the invention, particularly preferred pearlizing components are ethylene glycol distearate and polyethylene glycol distearate having 3 glycol units.

Moisture-donating substances are, for example, isopropyl palmitate, glycerol and/or sorbitol, which can be used in amounts by weight of from 0.1 to 50%.

Superfatting agents which can be used are substances such as, for example, lanolin and lecithin, nonethoxylated and polyethoxylated or acylated lanolin and lecithin derivatives, polyol fatty acid esters, mono-, di- and triglycerides and/or fatty acid alkanolamides.

Suitable preservatives are, for example, phenoxyethanol, parabens, pentanediol or sorbic acid.

Dyes which can be used are the substances approved and suitable for cosmetic purposes.

Suitable antidandruff agents or fungicidal active ingredients are, preferably ketoconazole, Climbazole® , Octopirox® , oxiconazole, terbinafine, bifonazole, butoconazole, cloconazole, clotrimazole, econazole, enilconazole, fenticonazole, isoconazole, miconazole, sulconazole, tioconazole, fluconazole, itraconazole, terconazole and naftifine, Zn pyrethione and octopyrox.

Biogenic active ingredients which can be used are, for example, Bisabolol® , Allantoin® , Phytantriol® , Panthenol® , AHAs, plant extracts and vitamin complexes.

The acids or alkalis used for adjusting the pH are preferably citric acid and/or sodium hydroxide solution.

The compositions are usually adjusted to a pH in the range from 2 to 12, preferably pH 3 to 8.

The examples and applications below serve to illustrate the invention in more detail, without, however, limiting it thereto (all percentages are percentages by weight).

Volumizing CONDITIONER

| A | Water | ad 100 |
| | Glycerol | 4.00 |

(continued)

|   |   |   |
|---|---|---|
| | Dissolvine Na2 (Akzo)<br>Ethylenediamine tetraacetate, Na | 4.00 |
| | Methyl parasept<br>Methylparaben (Nipa) | 0.30 |
| B | Incroquat SDQ25 (Croda)<br>Stearalkonium Chloride | 4.50 |
| | SilCare™ Silicone 31M50 (CLARIANT)<br>Caprylyl Trimethicone | 7.50 |
| | Incroquat Behenyl ™S (Croda)<br>Behentrimonium Methosulfate & Cetearyl Alcohol | 6.00 |
| | SilCare™ Silicone 140M30 (CLARIANT)<br>Caprylyl-Bis-PEG/PPG 20/20 Methyl ether Dimethicone | 4.00 |
| | Jeesorb O-20K (Jeen Int.) Polysorbate 80 | 0.50 |
| | Nipasol (Nipa)<br>Propylparaben | 0.10 |
| C | Crodacel QS (Croda)<br>PG Hydroxyethylcellulose Distearyldimethylammonium chloride | 2.50 |
| | Crodasone W (Croda)<br>Hydrolyzed wheat protein<br>Hydroxypropylpolysiloxane | 1.00 |

| PHASE D | | | |
|---|---|---|---|
| D-Panthenol | 1.00 | Panthenol | Jeen Int. |
| Fragrance | 0.30 | | |

Preparation method

I     Heat water to 70 - 75°C. Add the other constituents of phase A with slow stirring
II    Heat phase B to 70 - 75°C.
III   Slowly stir phase B into phase A
IV    Cool, with stirring, to 40°C
V     Add phase C and cool below 35°C
VI    Add panthenol and fragrance with slow stirring and stir for 3-5 minutes.

Hair gel

| A | Jaguar C-162 (Rhodia)<br>Hydroxypropyl Guar Hydroxypropyl Trimonium Chloride | 1.20 |
|---|---|---|
| | Kytamer PC (Amerchol)<br>Chitosan PCA | 0.20 |
| | Phenonip (CLARIANT)<br>Phenoxyethanol & Methylparaben<br>& Ethylparaben & Propylparaben<br>& Butylparaben & Isobutylparaben | 0.25 |

(continued)

| B | SilCare<sup>TM</sup> Silicone 140M30 (CLARIANT) Caprylyl-Bis-PEG/PPG 20/20 Methyl Ether Dimethicone | 1.00 |
|---|---|---|
| | Silcare Silicone 31 M60 (CLARIANT) Caprylyl Trimethicone | 0.50 |
| C | Water | ad 100 |
| | Uvinul MS40 (BASF) Benzophenone-4 | 0.10 |
| D | Jeesorb O-20 (Jeen Int.) Polysorbate 80 | 0.40 |
| | Fragrance | 0.25 |

Preparation method

I    Dry mix Jaguar and Kytamer. Disperse in water by vigorous mixing.
II   Heat phase A to 65 - 70°C; mix for 20 minutes
III  Add phase B and mix for 10 minutes
IV   Dissolve benzophenone-4 in water and add
V    Cool to below 35°C
VI   Dissolve fragrance in polysorbate and add

LEAVE-ON HAIR CONDITIONER

| A | Water | ad 100 |
|---|---|---|
| | Carbopol Ultrez 10 (B F Goodrich) Carbomer | 0.10 |
| | Pemulen TR-2 (B F Goodrich) Acrylates/$C_{10}$-30 Alkyl Acrylate Cross polymer | 0.30 |
| B | Silcare Silicone 41M15 (CLARIANT) Caprylyl Methicone | 0.90 |
| | Silcare Silicone 41M20 (CLARIANT) Lauryl Methicone | 2.10 |
| | SilCare™ Silicone 140M30 (CLARIANT) Caprylyl-Bis-PEG/PPG 20/20 Methyl Ether Dimethicone | 2.00 |
| | Finsolv TN (Finetex) $C_{12}$-15 Alkyl Benzoate | 0.50 |
| | Jeesorb O-20 (Jeen Int.) Polysorbate 80 | 0.40 |
| C | TEA (Dow Chem) Triethanolamine 99 % | 0.30 |
| D | DL Panthenol (Jeen Int.) | 0.20 |
| | Fragrance | 0.40 |
| | Timiron Starlight Green | 0.15 |

(continued)

| | | | |
|---|---|---|---|
| | | Hispagel Oil (Centerchem) Glycerol, Glyceryl Polyacrylate | 2.00 |
| | E | Water | 2.00 |
| | | Uvinul MS 40 (BASF) Benzophenone-4 | 0.01 |
| | | Flamenco Satin Pearl 3500 | 0.25 |
| | F | F D & C Blue # 1 | Q.S. |
| | | F D Red # 33 | q.s. |
| | | Phenonip (Clariant) Phenoxyethanol & Methylparaben & Ethylparaben & Propylparaben & Butylparaben & Isobutylparaben | 0.25 |

Preparation method

I      Dry mix Pemulen and Carbopol; disperse in water by vigorous stirring (30 - 45 minutes)

II     Mix constituents of phase B and add to A. Stir for 20 minutes.

III    Add TEA and stir at a higher speed.

IV    Add phase D with stirring.

V     Disperse Benzophenone-4 and Flamenco Satin Pearls in water and add.

VI    Add dye and fragrance with stirring and homogenize.

Care shampoo

| | | | |
|---|---|---|---|
| A | Water | | ad 100.00% |
| B | GENAMIN® STAC (Clariant) Steartrimonium Chloride | | 2.00% |
| | Cetyl alcohol | | 0.50 % |
| | Sodium chloride | | 0.50 % |
| | GENAPOL® PMS        (Clariant) Glycol Distearate | | 1.50 % |
| | Sodium cumenesulfonate | | 0.50 % |
| | Celquat® SC 230 M Polyquaternium-10 | | 0.30 % |
| | Glucamate DOE 120 PEG-120 Methyl Glucose Dioleate | | 2.50 % |
| | GENAPOL® LRO LIQUID        (Clariant) Sodium Laureth Sulfate | | 35.00 % |
| | HOSTAPON® KCG        (Clariant) Sodium Cocoyl Glutamate | | 8.00 % |
| C | GENAGEN® CAB 818        (Clariant) Cocamidopropyl Betaine | | 6.00 % |
| | Dye | | q.s. |
| | Preservative | | q.s. |
| | Fragrance | | 0.30 % |

(continued)

| | | |
|---|---|---|
| D-Panthenol | | 0.50 % |
| SILCARE® SILICONE 140M30 (Clariant)<br>Caprylyl-Bis-PEG/PPG 20/20 Methyl Ether Dimethicone | | 0.50 % |

Preparation method

I     Dissolve components B in A with stirring and heat to 65°C.
II    Cool to room temperature.
III   Add components C one after the other to II
IV    Adjust pH

Cream rinse

| A | Genamin® KDMP (Clariant)<br>Behentrimonium Chloride | 2.50 % |
|---|---|---|
| | Hostaphat® KL 340 D (Clariant)<br>Trilaureth-4 Phosphate | 1.50 % |
| | SilCare® Silicone 140M30 (Clariant)<br>Caprylyl-Bis-PEG/PPG 20/20 Methyl Ether Dimethicone | 0.50 % |
| | Cetyl Alcohol | 3.00 % |
| | Jojoba Oil | 3.00 % |
| B | Water | ad 100 % |
| | Preservative | q.s. |
| C | Fragrance | 0.30 % |
| | Dye solution | q.s. |

Preparation method

I     Melt A at about 75°C.
II    Heat B to about 75°C.
III   Add II to I with stirring and allow to cool with stirring.
IV    Add C to III at about 30°C.
V     Adjust to pH 4

Hair ends fluid

| A | SilCare® Silicone 140M30 (Clariant) Caprylyl-Bis-PEG/PPG 20/20 Methyl Ether Dimethicone | 59.90 |
|---|---|---|
| | SilCare® Silicone 41M15 (Clariant) Caprylyl Methicone | 20.00 |
| | Dow Corning® 345 Cyclomethicone | 10.00 |
| | Alcohol denat. | 10.00 |
| | Illipe Butter Shorea Stenoptera | 0.10 |

Preparation method

Mix the components and homogenize

**Claims**

1. A single-phase hair cosmetic preparation comprising, as active substance, a compound of the formula

$$R(OCHR'CH_2)_w(OCHR''CH_2)_x\text{-}O(CH_2)_3\text{-}Me_2SiO(R'''MeSiO)_y(Me_2SiO)_zSiMe_2(CH_2)_3\text{-}O\text{-}$$

$$(CH_2CHR''O)_x(CH_2CHR'O)_wR, \qquad \text{(formula 1)}$$

where Me is methyl, R is an alkyl group having 1 to 4 carbon atoms, R' and R", independently of one another, are hydrogen or an alkyl group having 1 to 4 carbon atoms, R'" is a linear or branched alkyl group having 4 to 18 carbon atoms, and x and w may be a number from 0 to 60, preferably 5 to 30, particularly preferably 10 to 20, y may be a number from 5 to 100 and z may be a number from 5 to 200, where the sum of the numbers x and w must be greater than or equal to 1 and the alkoxy groups may be in random distribution or else be in block form, y may be a number from 5 to 100 and z may be a number from 5 to 200.

2. A single-phase hair cosmetic preparation as claimed in claim 1, which comprises a compound of the formula 1 where R is methyl, R' is hydrogen, R" is methyl, R'" is n-octyl, w = 20, x = 20, y = 25 and z = 75, and also caprylyl-bis-PEG 10 methyl ether dimethicone according to the formula 1 where R is methyl, R' is hydrogen, R" is methyl, R'" is n-octyl, w = 10, x = 0, y = 25 and z = 75.

3. A single-phase hair cosmetic preparation as claimed in claim 1 based on water or water/alcohol, which comprises 0.01 to 30% of the compound of the formula 1.

4. A single-phase hair cosmetic preparation as claimed in claim 1 based on oil, which comprises 0.1 to 80% of the compound of the formula 1.

5. A single-phase hair cosmetic preparation as claimed in claim 1, which is an aqueous, aqueous-alcoholic or oil-containing preparation.

6. A single-phase hair cosmetic preparation as claimed in claim 1, which is a shampoo, hair conditioner, hair cure, hair rinse, volumizing spray, styling fluid, hair foam, hair gel, setting composition, hair spray, mousse, hair oil or split-end fluid.

EP 1 369 104 A1

## EUROPEAN SEARCH REPORT

**European Patent Office**

Application Number

EP 03 01 2563

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | DE 196 03 357 A (GEN ELECTRIC) 14 August 1996 (1996-08-14) * page 7, line 48 - page 11, line 4; claims 1,6 * | 1-6 | A61K7/075 A61K7/08 A61K7/11 |
| X | DE 199 07 587 A (BASF AG) 24 August 2000 (2000-08-24) * page 3, line 68 - page 5, line 7 * | 1-6 | |
| X | EP 0 670 342 A (GOLDSCHMIDT AG TH) 6 September 1995 (1995-09-06) * page 6, line 46 - page 7, line 6; claims 1-6 * | 1-6 | |
| D,A | EP 1 149 872 A (CLARIANT LIFE SCIENCE MOLECULE) 31 October 2001 (2001-10-31) * the whole document * | 1-6 | |

TECHNICAL FIELDS SEARCHED (Int.Cl.7)

A61K

The present search report has been drawn up for all claims

| Place of search | Date of completion of the search | Examiner |
|---|---|---|
| MUNICH | 23 September 2003 | Lindner, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

11

**ANNEX TO THE EUROPEAN SEARCH REPORT
ON EUROPEAN PATENT APPLICATION NO.**

EP 03 01 2563

This annex lists the patent family members relating to the patent documents cited in the above-mentioned European search report.
The members are as contained in the European Patent Office EDP file on
The European Patent Office is in no way liable for these particulars which are merely given for the purpose of information.

23-09-2003

| Patent document cited in search report | | | Publication date | Patent family member(s) | | Publication date |
|---|---|---|---|---|---|---|
| DE 19603357 | A | | 14-08-1996 | DE | 19603357 A1 | 14-08-1996 |
| | | | | FR | 2730497 A1 | 14-08-1996 |
| | | | | GB | 2297757 A ,B | 14-08-1996 |
| | | | | JP | 8319351 A | 03-12-1996 |
| | | | | US | 5684112 A | 04-11-1997 |
| | | | | US | 5817302 A | 06-10-1998 |
| DE 19907587 | A | | 24-08-2000 | DE | 19907587 A1 | 24-08-2000 |
| | | | | AT | 222746 T | 15-09-2002 |
| | | | | CA | 2363079 A1 | 31-08-2000 |
| | | | | DE | 50000418 D1 | 02-10-2002 |
| | | | | DK | 1154751 T3 | 14-10-2002 |
| | | | | WO | 0049998 A1 | 31-08-2000 |
| | | | | EP | 1154751 A1 | 21-11-2001 |
| | | | | ES | 2182784 T3 | 16-03-2003 |
| | | | | JP | 2002537315 A | 05-11-2002 |
| | | | | PT | 1154751 T | 31-01-2003 |
| EP 0670342 | A | | 06-09-1995 | DE | 4407189 A1 | 07-09-1995 |
| | | | | AT | 165106 T | 15-05-1998 |
| | | | | CA | 2142537 A1 | 05-09-1995 |
| | | | | CN | 1112945 A ,B | 06-12-1995 |
| | | | | DE | 59501875 D1 | 20-05-1998 |
| | | | | EP | 0670342 A1 | 06-09-1995 |
| | | | | ES | 2115278 T3 | 16-06-1998 |
| | | | | US | 5565194 A | 15-10-1996 |
| EP 1149872 | A | | 31-10-2001 | US | 6346553 B1 | 12-02-2002 |
| | | | | CA | 2344181 A1 | 13-10-2001 |
| | | | | EP | 1149872 A2 | 31-10-2001 |
| | | | | JP | 2002012668 A | 15-01-2002 |

For more details about this annex : see Official Journal of the European Patent Office, No. 12/82